# EUROPEAN PATENT APPLICATION

(11) **EP 1 537 846 A1**
(43) Date of publication of application: **08.06.2005**
(21) Application number: 03795361.9
(22) Date of filing: 10.09.2003
(51) Int. Cl.: A61J 3/02

(54) **PROCESS FOR THE PRODUCTION OF MICROSPHERES AND UNIT THEREFOR**

(30) Priority: 11.09.2002 JP 2002265468
(71) Applicant: TANABE SEIYAKU CO., LTD., Chuo-ku, Osaka-shi, Osaka 541-8505 (JP)
(72) Inventor: SUZUKI, Akira c/o Tanabe Seiyaku Co., Ltd., Osaka-shi Osaka 541-8505 (JP); TANIMOTO, Masahiko, Ashiya-shi, Hyogo 659-0014 (JP); MURATA, Junichi, Senboku-gun, Osaka 595-0813 (JP)
(74) Representative: HOFFMANN EITLE
(86) International application number: PCT/JP2003/011557
(87) International publication number: WO 2004/024056

(57) **Abstract**

In the method for preparation of microspheres by in-water drying method, an iterative process is employed, which comprises emulsifying a medicament-containing polymer solution (4) containing an organic solvent in an emulsifying device (1) to form an emulsion; transferring this emulsion into a microsphere storage tank (2); introducing a part of said emulsion to a cross flow filter (3) from the microsphere storage tank; and returning a liquid passing over the cross flow filter to the microsphere storage tank (2). Since a small amount of microsphere is repeatedly produced, this process permits the downsizing and airtight closing of an apparatus therefor, and further makes it possible to freely control the production scale of microsphere.

## Description

### TECHNICAL FIELD

The present invention relates to an efficient method for preparation of microsphere by a circulation process, and an apparatus for preparation of microsphere to be used in this method. In the method of the present invention, microsphere may efficiently be prepared by the circulation process comprising a combination of an emulsifying device, a microsphere storage tank and a cross flow filter.

### BACKGROUND ART

As a method for preparation of microsphere containing a medicament, various methods have been known, for example, in-water drying method, a phase-separation method, a spray drying method, a solvent-diffusion method, etc., and among the in-water drying methods, various methods are known, for example, methods using O/W emulsion (e.g., JP-A-4-46115, pages 1-6, JP-A-6-32732, pages 1-8), S/O/W emulsion (e.g., JP-A-8-151321, pages 10-15), W/O/W emulsion (e.g., JP-A-6-145046, pages 1-11, JP-A-9-221417, pages 1-11) or O/O/W emulsion (e.g., JP-A-6-211648, pages 1-8). Further, as a method for removing an organic solvent from these emulsions, a method by solvent evaporation has been known.

In the methods for evaporation of organic solvent from emulsion, the emulsion to be used for in-water drying method is generally prepared by adding an oil phase (O, S/O, W/O or O/O) into an aqueous phase, by which whole amount of emulsion as required is prepared at one time (e.g., JP-A-4-46115, pages 1-6, JP-A-6-145046, pages 1-11).

By the way, various emulsifying devices being required for the preparation of microsphere by in-water drying method have been known, for example, one being equipped within a batch tank for batch-treatment, one being equipped outside of an batch tank and being suitable for continuous treatment, etc. (e.g., Development in Chemical Industry, 24, Stirring/Mixing, published in 1990, Maki Shoten, page 187-191).

Further, in Handbook for applying skills of emulsifying/dispersion, published in 1987, Science Forum Inc., February 25, 1987, pages 140-143, pages 472-474, the microcapsule technique utilizing emulsion by in-water drying method is disclosed, and in said literature, the continuous mixing by a square mixer, one of static mixers, is also disclosed. There has been known an example for producing microsphere by forming an emulsion by continuous emulsification (e.g., JP-A-8-259460, page 12, USP 5945126, pages 1-12).

However, these methods for preparation of microsphere have problems, that is, for the large-scale production of microsphere, a large amount of emulsion should be subjected to in-water drying in one scoop and then the equipment for evaporation of organic solvent should also be enlarged.

Further, as a method for membrane filtration, there are a dead-end filtration where the total volume of a subject fluid is treated by a membrane, and a cross flow filtration where a subject fluid is flowed at right angle to a fluid penetrating through a membrane and thereby the subject fluid is partially filtered (cf., PDA Journal of Pharmaceutical Science & Technology, vol. 50, no. 4, p. 252-261 (1996)). The crossflow filtration is suitable for a large-scale treatment because a filter thereof is hardly clogged, and hence, it has been utilized in water-treatment system, etc., and further some examples have been known wherein a cross flow filtration is utilized in the collection and washing of microsphere prepared by in-water drying method (e.g., USP 6294204, pages 6-8, WO 96/35414, pages 11, 12).

Moreover, an apparatus has been proposed where the continuous phase of microsphere suspension obtained by the production of microsphere is replaced by water and a vehicle for drug formulation by filtration (e.g., USP 6270802, pages 1-10).

### DISCLOSURE OF INVENTION

An object of the present invention is to provide a method for preparation of microsphere having a high quality by in-water drying method, which is characterized by preparing microsphere by an iterative process, whereby an apparatus for preparation of microsphere is downsized, and the airtight closing of the whole apparatus is easily achieved in order to prevent the contamination of bacteria and the diffusion of organic solvent into the atmosphere which causes an environmental problem.

The present inventors have found that by repeating the small-scale preparation of microsphere, and using a process of accumulating microsphere thus obtained, an apparatus for preparation of microsphere may be downsized and microsphere of high quality can be obtained, and further they have found that a production scale of microsphere may be freely controlled, and finally they have accomplished the present invention. That is, the present invention relates to a method for preparation of microsphere comprising the following circulation steps:
(a) emulsifying a medicament-containing polymer solution containing a medicament, a biocompatible and biodegradable hardly-water-soluble polymer and an organic solvent having a boiling point lower than that of water into an aqueous solution in an emulsifying device to form an emulsion wherein said medicament-containing polymer solution is dispersed in the aqueous solution;
(b) transferring the obtained emulsion into a microsphere storage tank;
(c) introducing a part of the emulsion from the microsphere storage tank to a cross flow filter;
(d-1)-i) returning a liquid passing over the cross flow filter to the microsphere storage tank;
(d-1)-ii) recycling a filtrate filtered from the above cross flow filter as an aqueous solution for Step (a), repeating Steps (a) to (d-1), and when an organic solvent having a boiling point lower than that of water is immiscible with water, then evaporating off said organic solvent within the microsphere storage tank during this circulation procedure; or
(d-2)-i) returning a liquid passing over the cross flow filter to the microsphere storage tank;
(d-2)-ii) discharging a filtrate filtered from the above cross flow filter without recycling it as an aqueous solution for Step (a), repeating Steps (a) to (d-2) with using a fresh aqueous solution, and when the organic solvent having a boiling point lower than that of water is immiscible with water, then evaporating off said organic solvent within the microsphere storage tank during this circulation procedure;
(e) collecting microsphere in the microsphere storage tank after Step (d-1) or (d-2) is completed.

According to the method of the present invention, since only the aqueous solution is efficiently separated by cross flow filtration from the emulsion being prepared in the emulsifying device and accumulated in the microsphere storage tank, even if the emulsification step is repeated, the increase in the emulsion volume in the microsphere storage tank can be restrained, and further, the emulsification step is carried out in a small scale so that the emulsification is more uniformly achieved with ease, and microsphere of high quality can be prepared.

### BRIEF DESCRIPTION OF DRAWINGS

Fig. 1 shows a layout of an apparatus for production of microsphere, wherein an aqueous solution is recycled.
Fig. 2 shows a layout of an apparatus for production of microsphere, wherein an aqueous solution is not recycled.

### EXPLANATION OF SYMBOLS

(1) An emulsifying device
(2) A microsphere storage tank
(3) A cross flow filter
(4) A storage tank for medicament-containing polymer solution
(5) A storage tank for an aqueous solution

### BEST MODE FOR CARRYING OUT THE INVENTION

Each step of the method of the present invention is illustrated in more detail below.

The medicament-containing polymer solution for Step (a) containing a medicament, a biocompatible and biodegradable hardly-water-soluble polymer, and an organic solvent having a boiling point lower than that of water includes, for example, the following ones:
(i) A solution (O) wherein a biocompatible and biodegradable hardly-water-soluble polymer and a medicament are dissolved in an organic solvent having a boiling point lower than that of water;
(ii) A suspension (S/O) wherein a biocompatible and biodegradable hardly-water-soluble polymer is dissolved in an organic solvent having a boiling point lower than that of water, and a medicament is suspended in the obtained solution;
(iii) A dispersion (W/O) wherein a biocompatible and biodegradable hardly-water-soluble polymer is dissolved in an organic solvent having a boiling point lower than that of water, and an aqueous solution of a medicament is dispersed in the obtained solution;
(iv) A dispersion (O/O) wherein one of biocompatible and biodegradable hardly-water-soluble polymers is dissolved in an organic solvent having a boiling point lower than that of water, and in the obtained polymer solution is dispersed a solution of the other biocompatible and biodegradable hardly-water-soluble polymer in the same organic solvent, and a medicament is dissolved or suspended in the dispersed polymer solution;

In case that a solution (O) of the above (i) is prepared by dissolving a medicament in a polymer solution in an organic solvent, the concentration of the polymer in the polymer solution in an organic solvent may vary according to the kinds or molecular weight of said polymer, but it is usually in the range of 1 to 80 % by weight, preferably in the range of 20 to 60 % by weight. Further, it is preferable to dissolve a medicament in an amount of 0.1 to 40 % by weight to the weight of the polymer, and in order to improve the medicament content in microsphere, it is more preferable to dissolve a medicament in an amount of 1 to 30 % by weight in the polymer solution in an organic solvent.

In case that the solubility of a medicament in an organic solvent is low, then the medicament and the polymer are dissolved in a solvent system in which both can be soluble, and the solvent is evaporated tentatively to give a solid solution comprising the medicament and the polymer, and the obtained solid solution is further dissolved in a subject organic solvent to give an organic solvent solution (cf., USP 5556642/JP-A-6-32732).

In case that a suspension (S/O) of the above (ii) is prepared by suspending a medicament in a polymer solution in an organic solvent, it is preferable to suspend the medicament in an amount of 0.1 to 40 % by weight to the weight of the polymer in the same polymer solution in an organic solvent as the solution (O) of the above (i), and in order to improve the medicament content in microsphere, it is more preferable to suspend the medicament in an amount of 1 to 30 % by weight in the polymer solution in an organic solvent. When the medicament is suspended in the polymer solution in an organic solvent, then it is sufficient for the medicament to be insoluble in said organic solvent.

The medicament may be suspended in the polymer solution in an organic solvent by a homogenizer, a sonicator, etc., and it is preferable to emulsify the resulting suspension into an aqueous solution immediately after the medicament is suspended in the polymer solution in an organic solvent.

Further, when a medicament is suspended in the polymer solution in an organic solvent, said medicament may previously be pulverized in order to prevent an initial burst from microsphere, though the burst may depend on the particle size of microsphere to be produced, and the average particle size of the medicament should be in the range of 1/5 to 1/10000, more preferably in the range of 1/10 to 1/1000 of the average particle size of microsphere to be produced.

The pulverization of medicament to be suspended in the polymer solution in an organic solvent may be carried out by a conventional pulverization method such as by milling method, crystallization method, spray-drying method, etc.

In the milling method, the medicament may physically be pulverized by a conventional pulverizer, such as jet-mill, hammer mill, rotary ball-mill, vibratory ball-mill, beads mill, shaker mill, rod mill, tube mill, etc.

In the crystallization method, the medicament may be pulverized by dissolving once in a suitable solvent, precipitating by means of regulating the pH, changing the temperature, changing components of solvents, etc., and then collecting by filtration, centrifugation, etc.

In the spray drying method, the medicament may be pulverized by dissolving in a suitable solvent, spraying the resulting solution into a drying chamber of a spray drier using a spray nozzle to volatilize the solvent within the spray drops in a quite short time.

With respect to peptidic medicaments, the pulverization thereof should be carried out with maintaining its pharmacological activities, and it is preferably carried out, for example, by the following methods.
(A) A method of atomizing an aqueous solution containing a water-soluble high-molecular substance such as gelatin, etc. and a polypeptide by a spray drier (cf., JP-A-4-36233)
(B) A method of pulverization which comprises lyophilizing an aqueous solution containing a polypeptide and a water-soluble high-molecular substance and pulverizing the lyophilized resultant by a jet mil (JP-A-8-225454)
(C) A method of pulverization, which comprises adding an aqueous polypeptide solution into acetone to precipitate polypeptide fine particles (Journal of Encapsulation, vol. 14 (2), pages 225-241, 1997)
(D) A method of pulverization, which comprises mixing a surfactant and a polypeptide in water and rapidly drying the resulting mixture (JP-A-9-315997)
(E) A method of pulverization, which comprises adding a water-miscible organic solvent or a volatile salt into an aqueous polypeptide solution and lyophilizing the resultant (JP-A-11-322631)
(F) A method of pulverization, which comprises lyophilizing an aqueous mixture of a polypeptide and polyethyleneglycol and dissolving the polyethyleneglycol in an organic solvent (JP-A-11-302156)
(G) A method of pulverization, which comprises adding a water-miscible organic solvent which does not dissolve a polypeptide to a frozen product of an aqueous solution containing a polypeptide and a phase separation inducer to dissolve said phase separation inducer and ice contained in said frozen product, and collecting polypeptide fine particles from the resulting dispersion of polypeptide fine particles (WO 02/30449)

The method of dispersing an aqueous medicament solution in a polymer solution in an organic solvent to give the dispersion (W/O) of the above (iii) may be employed in cases where such a medicament is water-soluble, and said organic solvent for dissolving a polymer is immiscible with water, and particularly, this method is preferably employed to a medicament having a distribution ratio in n-octanol/water of not more than 0.1.

The concentration of medicament in the aqueous medicament solution is usually 0.1 % by weight or more (less than the solubility of said medicament), and more preferably 1 % by weight or more. Further, it is preferable to disperse the aqueous medicament solution in an amount of 0.1 to 30 % by weight, more preferably in an amount of 1 to 20 % by weight, in the same polymer solution in an organic solvent as the solution (O) of the above (i).

The aqueous medicament solution may additionally contain, in addition to the medicament, other additives, for example, stabilizers (e.g., albumin, gelatin, 4 sodium ethylenediaminetetraacetate, dextrin, sodium hydrogen sulfite, polyethyleneglycol, etc.), preservatives (e.g., p-hydroxybenzoic acid esters (methyl ester, ethyl ester, propyl ester, butyl ester), etc.), pH adjusters (e.g., carbonic acid, acetic acid, oxalic acid, citric acid, phosphoric acid, hydrochloric acid, sodium hydroxide, or a salt of these acids such as sodium carbonate, sodium hydrogen carbonate, etc.).

When a peptidic medicament is used, an aqueous medicament solution thereof may additionally contain a medicament-retaining substance such as gelatin, agar powder, polyvinyl alcohol, basic amino acids (e.g., arginine, histidine, lysine, etc.)).

When an aqueous medicament solution is dispersed in a polymer solution in an organic solvent, the average particle size of liquid droplets of the aqueous medicament solution should be, depending on the particle size of microsphere, in the range of 1/5 to 1/10000, more preferably in the range of 1/10 to 1/1000 of the average particle size of microsphere to be produced, and the dispersion procedure is preferably carried out by using a homogenizer, a sonicator, etc. Further, it is preferable to emulsify the resulting dispersion into an aqueous solution immediately after the medicament is dispersed in a polymer solution in an organic solvent.

In case that the dispersion (O/O) of the above (iv) is prepared, a medicament is dissolved or suspended in one of polymer solutions in a similar manner to the preparation of the solution (O) of the above (i) or the suspension (S/O) of the above (ii), and the resulting solution or suspension is dispersed in another polymer solution which is immiscible with said solution or suspension in a similar manner to the preparation of the dispersion (W/O) of the above (iii). Either of the organic solvents in the polymer solutions may be the same ones as those for the solution (O) of the above (i).

The biocompatible and biodegradable hardly-water-soluble polymer may be any biocompatible and biodegradable hardly-water-soluble polymer which is usually used in the pharmaceutical field. In the present invention, the hardly-water-soluble polymer means ones, which requires 1000 g or more of water for dissolving 1 g of said polymer at 25°C.

The biocompatible and biodegradable hardly-water-soluble polymer includes, for example, a polyester of hydroxyfatty acid, poly-α-cyanoacrylic acid ester, polyamino acid, etc. Among them, the polyester of hydroxyfatty acid is preferably ones having an average molecular weight of 2000 to 800000, more preferably ones having an average molecular weight of 5000 to 200000, and most preferably ones having an average molecular weight of 5000 to 50000.

Specific examples of the polyester of hydroxyfatty acid are polylactic acid, lactic acid-glycolic acid copolymer, 2-hydroxybutyric acid-glycolic acid copolymer, poly-β-hydroxyburyric acid, etc. The lactic acid-glycolic acid copolymer preferably has a molar ratio of lactic acid/glycolic acid in the range of 90/10 to 30/70, and more preferably in the range of 80/20 to 40/60, and the 2-hydroxybutyric acid-glycolic acid copolymer preferably has a molar ratio of 2-hydroxybutyric acid/glycolic acid in the range of 90/10 to 30/70, and more preferably 80/20 to 40/60.

The organic solvent having a boiling point lower than that of water means an organic solvent having a boiling point lower than that of water under the same pressure, and may include either of water-miscible ones or water-immiscible ones.

The water-miscible organic solvent having a boiling point lower than that of water means ones having a boiling point lower than that of water and being able to be completely miscible with water at any ratio, for example, water-miscible ketone solvents (e.g., acetone, etc.), water-miscible ether solvents (e.g., tetrahydrofuran, etc.), nitrile solvents (e.g., acetonitrile, etc.), and acetone is more preferable.

The water-immiscible organic solvent having a boiling point lower than that of water means ones having a boiling point lower than that of water but being miscible with water only at a ratio of 10 % by volume or less, for example, halogenated aliphatic hydrocarbon solvents (e.g., methylene chloride, chloroform, carbon tetrachloride, chloroethane, dichloroethene, trichloroethane, etc.), aliphatic ester solvents (e.g., ethyl acetate, etc.), aromatic hydrocarbon solvents (e.g., benzene, etc.), aliphatic hydrocarbon solvents (e.g., n-hexane, n-pentane, cyclohexane, etc.), water-immiscible ether solvents (e.g., diethyl ether, diisopropyl ether, methyl isobutyl ether, methyl tert-butyl ether, etc.), and halogenated aliphatic hydrocarbon solvents, aliphatic ester solvents may be preferable, and methylene chloride, chloroform, and ethyl acetate are more preferable.

The medicaments to be applied to the method of the present invention include, for example, antitumor agents, peptidic medicaments, antibiotics, antipyretics, analgesics, antiinflammatories, antitussives, expectorants, sedatives, muscle relaxants, antiepileptics, antiulcers, antidepressants, antiallergic agents, cardiotonics, antiarrythmic agents, vasodilators, antihypertensive diuretics, antidiabetics, antihyperlipidemic agents, anticoagulants, hemostatics, antitubercular agents, hormones, antinarcotic agents, bone resorption inhibitors, promoters of osteogenesis, promoters of fracture healing, agents for treatment of chondropathy, antiangiogenetics, antiemetics, etc.

Antitumor agents includes, for example, paclitaxel, bleomycin, methotrexate, actinomycin D, mitomycin C, vinblastine sulfate, vincristine sulfate, daunorubicin, doxorubicin, neocercinostatin, cytosine arabinoside, fluorouracil, tetrahydrofuryl-5-fluorouracil, krestin, picibanil, lentinan, tamoxifen, levamisole, bestatin, azimexon, , cisplatin, carboplatin, irinotecan hydrochloride, etc.

Peptidic medicament includes, for example, insulin, somatostatin, sandostatin, growth hormone, prolactin, adrenocortical tropic hormone (ACTH), ACTH derivatives, melanocyte stimulating hormone (MSH), thyrotrophin releasing hormone (TRH), thyroid stimulating hormone (TSH), luteinizing hormone (LH), luteinizing hormone releasing hormone (LHRH) and its derivatives, follicle stimulating hormone (FSH), vasopressin, desmopressin, oxytocin, calcitonin, elcatonin, parathyroid hormone (PTH), glucagons, gastrin, secretin, pancreozymin, cholecystokinin, angiotensin, human placental lactogen, human chorionic gonadotropin (HCG), enkephalin, enkephalin derivatives, endorphin, kyotorphin, interferons (e.g., α-, β-, γ-, etc.), interleukins (e.g., 1, 2, 3, 4, 5, 6, 7, 8, 9, 10, 11, 12, etc.), taftsin, thymopoietin, thymosin, thymostimulin, thymic humoral factor (THF), serum thymic factor (FTS) and its derivatives, and other thymic factors, tumor necrosis factor (TNF), chemokines and its derivatives, mini-cytokines and its derivatives, colony stimulating factors (e.g., CSF, GCSF, GMCSF, MCSF, etc.), motilin, dinorphin, bombesin, neurotensin, cerulein, bradykinin, urokinase, asparaginase, kallikrein, substance P, insulin-like growth factor (IGF-I, IGF-II), nerve growth factor (NGF), cell growth factors (e.g., EGF, TGF-α, TGF-β, PDGF, FGF hydrochloride, basic FGF, etc.), bone morphogenetic protein (BMP), neurotrophic factors (e.g., NT-3, NT-4, CNTF, GDNF, BDNF, etc.), blood coagulation factors VIII and IX, lysozyme chloride, polymixin B, colistin, gramicidin, bacitracin, erythropoietin (EPO), thrombopoietin (TPO), etc.

Antibiotics include, for example, gentamycin, dibekacin, kanendomycin, lividomycin, tobramycin, amikacin, fradiomycin, sisomicin, tetracycline hydrochloride, oxytetracycline hydrochloride, rolitetracycline, doxycycline hydrochloride, ampicillin, piperacillin, ticarcillin, aspoxicillin, cephalothin, cephaloridine, cefotiam, cefsulodin, cefmenoxime, cefmethazole, cefazolin, cefotaxime, cefoperazone, ceftizoxime, moxolactam, thienamycin, sulfazecin, azthreonam, etc.

Antipyretics, analgesics and anti-inflammatory agents include, for example, salicylic acid, sulpyrine, flufenamic acid, diclofenac, indomethacin, morphine, pethidine hydrochloride, levorphanol tartrate, oxymorphone, etc.

Antitussives and expectorants include, for example, ephedrine hydrochloride, methylephedrine hydrochloride, noscapine hydrochloride, codeine phosphate, dihydrocodeine phosphate, alloclamide hydrochloride, chlophedianol hydrochloride, picoperidamine hydrochloride, cloperastine, protokyrol hydrochloride, isoproterenol hydrochloride, salbutamol sulfate, terbutaline sulfate, etc.

Sedatives include, for example, chlorpromazine, prochlorperazine, trifluoperazine, atropine sulfate, methylscopolamine bromide, etc.

Muscle relaxants include, for example, pridinol methanesulfonate, tubocurarine chloride, pancuronium bromide, etc.

Antiepileptics include, for example, phenytoin, ethosuximide, sodium acetazolamide, chlordiazepoxide, etc.

Antiulcers include, for example, metoclopromide, histidine hydrochloride, etc.

Antidepressants include, for example, imipramine, clomipramine, noxiptiline, phenelzine sulfate, etc.

Antiallergic agents include, for example, diphenhydramine hydrochloride, chlorpheniramine maleate, tripelenamine hydrochloride, methdilazine hydrochloride, clemizol hydrochloride, diphenylpyraline hydrochloride, methoxyphenamine hydrochloride, etc.

Cardiotonics include, for example, trans-π-oxocamphor, theophylol, aminophylline, etilefrine hydrochloride, etc.

Antiarrythmic agents include, for example, azimilide, propranolol, alprenolol, bufetorol, oxyprenolol, etc.

Vasodilators include, for example, oxyfedrine hydrochloride, diltiazem hydrochloride, tolazoline hydrochloride, hexobendine, bamethan sulfate, etc.

Antihypertensive diuretics include, for example, hexamethonium bromide, pentrilium, mecamylamine hydrochloride, ecarazine hydrochloride, clonidine, etc.

Antidiabetics include, for example, glymidine sodium, glypizide, phenformin hydrochloride, buformin hydrochloride, metformin, etc.

Antihyperlipidemic agents include, for example, mevalotin, pravastatin sodium, simvastatin, fluvastatin, clinofibrate, clofibrate, simfibrate, bezafibrate, etc.

Anticoagulants include, for example, heparin sodium, etc.

Hemostatics include, for example, thromboplastin, thrombin, menadione sodium bisulfite, acetomenaphthone, ε-aminocaproic acid, tranexamic acid, carbazochrome sodium sulfonate, adrenochrome monoaminoguanidine methanesulfonate, etc.

Antitubercular agents include, for example, isoniazide, ethambutol, p-aminosalicylic acid, etc.

Hormones include, for example, prednisolone, prednisolone sodium phosphate, dexamethasone sodium hydrochloride, hexestrol phosphate, methimazole, etc.

Antinarcotic agents include, for example, levallorphan tartrate, nalorphine hydrochloride, naloxone hydrochloride, etc.

Bone resorption inhibitors include, for example, ipriflavone, alendronate, tiludronate, etc.

Promoters of osteogenesis include, for example, polypeptides such as bone morphogenetic protein (BMP), parathyroid hormone (PTH), cell growth factors (TGF-β, etc.), insulin-like growth factor (IGF-I, etc.), etc.

Promoters of fracture healing and agents for treatment of chondropathy include, for example, phosphodiesterase-4 inhibitors (PCT/JP02/04930, PCT/JP02/04931), etc.

Antiangiogenetics include, for example, angiogenesis suppressing steroids, fumagillin, fumagillol derivatives, angiostatin, endostatin, etc.

Antiemetics include, for example, 5-hydroxytryptamine type 3 receptor antagonists such as ondansetron or tropisetron, neurokinin 1 receptor antagonists, etc.

The medicaments referred to the above may be in the free form or in the form of a pharmaceutically acceptable salt. For example, when the medicament possesses a basic group such as an amino group, it may be used in the form of a salt with an inorganic acid (e.g., hydrochloric acid, sulfuric acid, nitric acid, etc.) or with an organic acid (e.g., carbonic acid, succinic acid, etc.). When the medicament possesses an acidic group such as a carboxyl group, it may be used in the form of a salt with an inorganic base (e.g., alkali metals such as sodium, potassium, etc.) or with an organic base (e.g., organic amines such as triethylamine, basic amino acids such as arginine, etc.).

Moreover, when the medicament forms a salt and hence the rate of uptake thereof into microsphere is low, said medicament may first be converted into a free form. When an acid addition salt is converted into a free form, it may be treated with a basic aqueous solution (e.g., an aqueous solution of an alkali metal hydrogen carbonate, an aqueous solution of an alkali metal carbonate, an aqueous solution of an alkali metal hydroxide, an aqueous solution of an alkali metal phosphate, an aqueous solution of an alkali metal hydrogen phosphate, a weakly basic buffer), and extracting with an organic solvent. When a base addition salt is converted into a free form, it may be treated with a weakly acidic aqueous solution (e.g., an aqueous ammonium chloride solution, a weakly acidic buffer, etc.) and extracting with an organic solvent. By evaporating the solvent from the extract by a conventional method, the medicament in a free form may be obtained.

Taking into consideration the properties of the medicament (e.g., a solubility in an organic solvent having a boiling point lower than that of water, a stability in an organic solvent, or a water-solubility), a dissolution profile of the medicament from microsphere, the content of the medicament or particle size thereof, etc., it may be decided if the medicament should be dissolved or suspended in a polymer solution in an organic solvent, or if an aqueous solution of the medicament should be dispersed in a polymer solution in an organic solvent. When the medicament may easily be denatured in an organic solvent, then the medicament may be suspended in the polymer solution in an organic solvent, or an aqueous solution of the medicament may be dispersed in the polymer solution in an organic solvent.

The emulsifying device to be used in the emulsification of a medicament-containing polymer solution into an aqueous solution may include known emulsifying devices, for example, a propeller stirrer, a turbine impeller mixer, a high-pressure emulsifier, an ultrasonic dispersion mixer, a static mixer, a high-speed rotary homogenizer utilizing inner shear (liquid-liquid shear), etc. By using a high-speed rotary homogenizer utilizing inner shear (liquid-liquid shear) (e.g., Clearmix manufactured by M Technique, Inc., Highsheer Inline Mixer manufactured by Silverson Machines, Inc., etc.), the emulsification strength may be increased, and hence, even if a medicament-containing polymer solution having a high viscosity is used, liquid droplets having a small particle size are formed within an aqueous solution, and microspheres having a small particle size may be produced.

The emulsification may be carried out by either batch-treatment or continuous treatment. The emulsification by batch treatment is carried out by T.K. AGI HOMO MIXER, T.K. COMBI MIX, T.K. Homo Jettor, Clearmix continuous batch or batch system of M Technique, Inc. On the other hand, the continuous emulsification is carried out by a high-speed sheer-type disperser and emulsifier (e.g., T.K. Homomic Line Flow manufactured by Tokushu Kika Kogyo Co., Ltd.), inline-type mixers (e.g., T.K. Pipeline Homo Mixer manufactured by Tokushu Kika Kogyo Co., Ltd., High-sheer Inline Mixer manufactured by Silverson Machines, Inc., Clearmix continuous system manufactured by M Technique, Inc., Square Mixer), etc.

In case of continuous emulsification, the obtained emulsion is preferably transferred to a microsphere storage tank continuously, and in case of emulsification by batch-treatment, the obtained emulsion is transferred to a microsphere storage tank in separate batches.

When the emulsification is carried out by batch-treatment, the capacity of the emulsifying device is preferably in the range of 1/1000 to 1/10 of the capacity of the microsphere storage tank, and the emulsification by batch-treatment is preferably carried out within 30 minutes, preferably within 10 minutes. On the other hand, in cases of continuous emulsification, the average retention time in the emulsifying device is preferably within 10 minutes, more preferably within 5 minutes.

The aqueous solution used is different in cases where a water-miscible organic solvent or a water-immiscible organic solvent is used as an organic solvent for polymer solution.

When a water-miscible organic solvent is used as an organic solvent for polymer solution, for example, as disclosed in WO 01/80835, a uniform solution containing water and a solvent which is immiscible with a water-miscible organic solvent and does not dissolve the polymer is preferably employed, and said uniform solution may contain a monovelent alcohol having 1 to 4 carbon atoms. In this case, a uniform mixture solution such as water-glycerin, aqueous ethanol-glycerin, etc. is preferable.

The concentration of the solvent which is immiscible with a water-miscible organic solvent and does not dissolve a polymer in an aqueous solution is in the range of 25 to 95 % by weight, preferably in the range of 50 to 90 % by weight, and more preferably in the range of 60 to 80 % by weight.

Further, the aqueous solution may further contain an emulsion stabilizer, and the emulsion stabilizer includes, for example, polyvinyl alcohol, polyvinylpyrrolidone, methyl cellulose, hydroxypropyl cellulose, gum arabic, chitosan, gelatin, lecithin, serum albumin, a nonionic surfactant (e.g., polyoxyethylene sorbitan fatty acid esters (Tween 80, Tween 60, manufactured by Nikko Chemicals Co., Ltd.), polyoxyethylene castor oil derivatives (HCO-60, HCO-50, manufactured by Nikko Chemicals Co., Ltd.)). The emulsion stabilizer is preferably added to the aqueous solution in an amount of 0.001 to 10 % by weight, preferably in an amount of 0.01 to 2 % by weight.

On the other hand, when a water-immiscible organic solvent is used as an organic solvent for polymer solution, the aqueous solution may be purified water, and if necessary, may contain an emulsion stabilizer. The same emulsion stabilizer may be used in the same amount as in case where a water-miscible organic solvent is used.

In the present method, emulsification in a large scale is not necessarily carried out at one time, and hence, the emulsifying device can be downsized. In addition, since the emulsifying device can be downsized, the agitation resistance can also be minimized so that the emulsification degree of the medicament-containing polymer solution and the aqueous solution is easily made uniform, and the variation in the particle size of liquid droplets of the emulsion is small and the emulsion containing liquid droplets having a small particle size can be formed. Further, the emulsification time can be shortened, and hence, the leakage of the medicament into the aqueous solution may be suppressed.

Further, in the present method, the emulsification is carried out by either batch-treatment or continuous treatment. In emulsification by batch-treatment, there is less variation in the emulsification time of a medicament-containing polymer solution and an aqueous solution than in emulsification by continuous treatment, and the emulsification speed is easily controlled with monitoring the particle size of liquid droplets, and hence, there is less variation in the particle size of liquid droplets, and further the emulsion containing liquid droplets having a small particle size may be more easily formed. Further, since an aqueous solution is replaced by a fresh one at every emulsification step, the concentration of organic solvent in an aqueous phase of the emulsion may be kept at a certain level or below during the emulsification procedure, which is desirable because the deterioration in quality such as the decrease in the medicament content, the variation in the particle size, etc., may be suppressed.

In both of continuous treatment and batch-treatment, the volume of the aqueous solution to be emulsified is preferably in the range of 10 to 300 times of the volume of the medicament-containing polymer solution, and in case of continuous emulsification, the volume of the aqueous solution is calculated based on the ratio of the volume of the aqueous solution to be introduced into the emulsifying device during the emulsification procedure and the volume of the medicament-containing polymer solution.

When the ratio of the polymer solution is lowered, some of the organic solvent leaks out into the aqueous solution from the liquid droplets of the medicament-containing polymer solution during the emulsification stage, and as a result, the liquid droplets may easily be solidified to some extent during the emulsification stage.

After the organic solvent gradually leaks out into the aqueous solvent or is evaporated, the liquid droplets in the emulsion just after the emulsification are solidified in the emulsifying device and the microsphere storage tank to give microsphere. In the microsphere formation stage, the term "liquid droplets" also includes microspheres in process of formation, but the average particle size of the above liquid droplets to be compared with the average particle size of microsphere means the particle size of liquid droplets just after the emulsification.

The emulsion having a medicament-containing polymer solution dispersed in an aqueous solution, which is obtained by emulsifying a medicament-containing polymer solution into an aqueous solution, includes, for example, the following emulsions.
(i) An emulsion (O/W), wherein a solution in which a biocompatible and biodegradable hardly-water-soluble polymer and a medicament are dissolved in an organic solvent having a boiling point lower than that of water is further dispersed in an aqueous solution;
(ii) An emulsion (S/O/W), wherein a suspension in which a biocompatible and biodegradable hardly-water-soluble polymer is dissolved in an organic solvent having a boiling point lower than that of water, and a medicament is suspended in the obtained solution is further dispersed in an aqueous solution;
(iii) An emulsion (W/O/W), wherein a dispersion in which a biocompatible and biodegradable hardly-water-soluble polymer is dissolved in an organic solvent having a boiling point lower than that of water, and an aqueous solution of a medicament is dispersed in the obtained solution is further dispersed in an aqueous solution;
(iv) An emulsion (O/O/W), wherein a dispersion in which one of biocompatible and biodegradable hardly-water-soluble polymers is dissolved in an organic solvent having a boiling point lower than that of water, and in the obtained polymer solution is dispersed a solution of the other biocompatible and biodegradable hardly-water-soluble polymer in the same organic solvent, and a medicament is dissolved or suspended in the dispersed polymer solution is further dispersed in an aqueous solution;

The emulsion storage tank to be used in Step (b) is preferably ones being made of a material which does not have a reactivity to the emulsion and the components of the emulsion, for example, ones being made of stainless-steel or Teflon, ones being coated with Teflon, ones being lined with glass, etc.

When the organic solvent having a boiling point lower than that of water is immiscible with water, the emulsion storage tank should have a function of evaporation of organic solvent, but when the organic solvent having a boiling point lower than that of water is miscible with water, the organic solvent is dissolved in the aqueous solution, and hence, the emulsion storage tank does not necessarily have a function of evaporation of organic solvent but may optionally have a function of evaporation of organic solvent. In either case, if the organic solvent leaks out into the aqueous solution during the emulsification stage and the liquid droplets of the medicament-containing polymer solution are solidified to some extent, then the emulsion may possibly be subjected to cross flow filtration without evaporating the organic solvent therefrom.

The function of evaporation of organic solvent may be one utilizing, for example, (A) a method of evaporation of organic solvent by a combination of warming, reduction in pressure, etc., (B) a method of blowing a gas around the liquid surface and controlling the contact area of an outer aqueous phase and a gaseous phase, and the rate of circulation and stirring speed of emulsion (JP-A-9-221418); (C) a method of rapidly evaporating an organic solvent with a hollow fiber membrane module (WO 01/83594), etc.

The hollow fiber membrane module is preferably, for example, a silicon-rubber pervaporation membrane (particularly a pervaporation membrane made of polydimethylsiloxane), a membrane prepared by filling silicon rubber into porous polytetrafluoroethylene (cf., JP-A-5-15749, etc.), a pervaporation membrane such as polyvinyl alcohol mixed membrane (cf., Chemical Engineering, March 1998, pp. 25-29). Specific examples of hollow fiber membrane module are a silicone membrane module ("NAGASEP" manufactured by Nagayanagi Kogyo Kabushiki Kaisha), a deaerating membrane element (SG-100 series, manufactured by Toray Industries, Inc.), a triple layer composite hollow fibers membrane (a deaerating membrane module, manufactured by Mitsubishi Rayon Co., Ltd.), a hollow fiber membrane module ("SEPAREL", manufactured by Dainippon Ink and Chemicals Inc.).

For the uniformity of the content in the microsphere storage tank and the introduction of a part of said content into the cross flow filter, the microsphere storage tank is preferably equipped with a stirring piece for flowing the emulsion such as a stirring blade or a magnetic stirrer, or a pump for suctioning a part of the emulsion from the lower part of the emulsion and returning it to the upper part of the emulsion.

The cross flow filtration in Step (c) is a filtration method wherein a subject emulsion is flowed in parallel with the membrane filter and a part of the liquid components of the subject is penetrated through the membrane filter, and a part of the aqueous solution in the emulsion flowing in parallel with the membrane filter is filtered, and penetrated as a filtrate into the opposite side of the membrane filter, and the remainder of the emulsion is flowed in parallel with the membrane filter. Since the direction of the flow of the emulsion is in parallel with the membrane filter, the clogging of the membrane filter seldom occurs and the decrease in the filtration efficiency is well suppressed.

The membrane filter is preferably one having a pore size of 1/300 to 1/3 of the average particle size of the desired microsphere, and usually ones having a pore size of 0.01 to 10 µm. The membrane filter to be used for the cross flow filtration is preferably one having a filtration membrane area of 0.001 to 0.1 m² per 1 liter of the capacity of the microsphere storage tank.

The cross flow filter is preferably ones wherein a membrane filter formed with a polymer such as polyvinylidene fluoride, regenerated cellulose, polyether sulfone, hydrophilic polyether sulfone, polyamide composite membrane, etc., is laminated to a flat plate form, or combined to a form of bundles of a fine cylinder so as to increase the surface area per unit volume, and specific examples thereof are Prostak manufactured by Millipore Corporation, Sartocon manufactured by Sartorius K. K., Ulticlean manufactured by Pall Corporation, Microflow manufactured by Cuno, Ltd., etc.

For the cross flow filtration, it is preferable to adjust the filtration speed of the filtrate from the cross flow filter to 1/100 to 1 /3 of the introducing speed of the emulsion into said filter.

In Step (d-1) and Step (d-2), a liquid passing over the surface of the cross flow filter without penetrating through the membrane filter thereof is returned to the microsphere storage tank. This passing liquid is one wherein the filtrate is removed from the emulsion to be introduced into the cross flow filter, and hence, the volume of the emulsion is decreased by the volume of the filtrate by cross flow filtration.

In Step (d-1), the filtrate is recycled as an aqueous solution for Step (a), and this filtrate and the medicament-containing polymer solution are subjected to emulsification, and thereafter, Step (b) to Step (d-1) are repeated. If necessary, the organic solvent having a boiling point lower than that of water is evaporated off from the filtrate, and then the resultant is used as an aqueous solution for Step (a). This evaporation of organic solvent is usually carried out in a pathway for connecting the filter and the emulsifying device, having a suitable equipment for evaporation of organic solvent.

As explained above, since the filtrate is recycled as an aqueous solution for the emulsification procedure, the microspheres to be produced by solidifying the liquid droplets of the medicament-containing polymer solution steadily accumulate in the tank in proportion to the number of emulsification. Therefore, as compared to the large-scale production of microsphere at one time, the production of microspheres in an industrial scale using a small-scale emulsifying device and a small-scale microsphere storage tank may be made possible.

In addition, according to the present method, in addition to the downsizing of the apparatus for preparation of microsphere, the construction and maintenance of a closed system may also easily be permitted by downsizing of the apparatus for preparation of microsphere, so that the contamination of bacteria from the outside of the apparatus, or the diffusion of organic solvent into the atmosphere can be prevented, and further, the required amount of microspheres may be produced only by controlling the number of emulsification procedure.

Moreover, when an aqueous solution contains an emulsion stabilizer, the emulsion stabilizer contained in the aqueous solution is also recycled, and hence, the consumption thereof may be reduced, and even if a medicament leaks out into the aqueous solution in the emulsification stage, etc., the medicament remains in the aqueous solution to be recycled, and hence, it may be possible to recover the medicament from the aqueous solution after the collection of microsphere.

On the other hand, in Step (d-2), the filtrate is not recycled as an aqueous solution for Step (a), and a fresh aqueous solution and the medicament-containing polymer solution are subjected to emulsification, and Step (b) to Step (d-2) are repeated.

In this case, the rate of emulsification is adjusted so as to transfer the substantially same amount of the emulsion into the microsphere storage tank as the amount of the filtrate to be recycled, and as a result, the amount of the emulsion in the microsphere storage tank is kept substantially constant, by which the capacity of the microsphere storage tank may be downsized. Further, by repeating Step (a) to Step (d-2), the microspheres, which are produced by solidification of liquid droplets of the medicament-containing polymer solution, accumulate in proportion to the number of emulsification procedure, by which the production of microspheres in an industrial scale using a small-scale emulsifying device and a small-scale microsphere storage tank may be made possible in a similar manner to Step (d-1).

Further, the construction and maintenance of a closed system of the apparatus may also be easily achieved, so that the contamination of bacteria from the outside of the apparatus, or the diffusion of organic solvent into the atmosphere can be prevented, and further, the production amount of microsphere may be easily controlled. Further, since an aqueous solution having the same components being previously prepared can be used as an aqueous solution for emulsification, the uniformity during the emulsification procedure may be maintained more easily as compared to cases where the filtrate is recycled as an aqueous solution.

As mentioned above, the filtrate to be obtained during the introduction into the cross flow filter in Step (c) may be recycled as an aqueous solution as in Step (d-1), or may be discharged without recycling as in Step (d-2). When it is recycled, and the organic solvent having a boiling point lower than that of water is miscible with water, said organic solvent contained in the recycled filtrate may be additionally evaporated during the circulation. By this evaporation, the content of the organic solvent in the aqueous solution is further reduced to promote the removal of the solvent and the formation of emulsion.

On the other hand, when the organic solvent having a boiling point lower than that of water is immiscible with water, the organic solvent is evaporated within the microsphere storage tank during the circulation, but when the evaporation of the organic solvent is not completed enough, prior to the collection of microspheres in Step (e), the organic solvent is supplementarily evaporated from microspheres by continuing the evaporation of the organic solvent in the microsphere storage tank with stopping the cross flow filtration after the completion of Step (d-1) or (d-2). In addition, when the evaporation of the water-immiscible organic solvent in the microsphere storage tank is not completed enough as mentioned above and the complete evaporation of the organic solvent is required from a view point of the microsphere formation or the regulation on residual organic solvent, then, in addition to the evaporation of organic solvent in the microsphere storage tank, another possible step for improving the solvent removal efficiency may be additionally taken. The evaporation of organic solvent from microsphere is additionally supplemented, for example, in Step (d-1), the cross flow filtration is continued while the organic solvent in the filtrate is evaporated, and then the filtrate after the evaporation is passed through without stopping at the emulsifying device and without emulsification procedure, or a procedure of returning it into the microsphere storage tank via a pipe being equipped separately is continued, or in Step (d-2), the cross flow filtration is continued while a fresh aqueous solution in an amount corresponding to the amount of the filtrate is introduced into the microsphere storage tank.

By the way, the preparation of microsphere by the method of the present invention is carried out until the desired amount of microsphere accumulates in the microsphere storage tank by repeating Step (a) to Step (d-1) or Step (d-2), and the completion point thereof may vary according to the capacity of said storage tank, or the desired amount of microsphere, but it is not desirable to store the produced microsphere in the storage tank for a long time in view of the quality control of microsphere, and hence, the treatment time required for the microsphere production is preferably within 2 days, more preferably within 1 day. Then, microspheres thus obtained are collected in Step (e).

In Step (e), microspheres may be collected from a suspension accumulated in the microsphere storage tank by filtration (cross flow filtration, dead-end filtration, etc.), centrifugation, etc.

When microspheres are collected by cross flow filtration, microspheres are efficiently collected by removing an aqueous solution in the suspension utilizing the same cross flow filter used for the production of microspheres. Further, by introducing a washing solution into the microsphere storage tank and circulating it into the cross flow filter, the collected microspheres are washed by utilizing the cross flow filtration. Thus, not only the preparation procedure of microspheres, but also the collection and washing thereof are made possible in a closed system.

In order to obtain microspheres having a desired particle size during the collecting procedure, the particle size of microsphere is further arranged by passing through a screen having a suitable opening, and after passing through a screen having an opening of 150 µm to 5 µm, the microsphere are preferably used as an injection.

Depending on the degree of evaporation of organic solvent, the organic solvent may occasionally remain in microspheres obtained by the present method, and the residual organic solvent may possibly be evaporated by the following methods.
(I) a method of warming collected microsphere in an aqueous phase at a temperature of a boiling point or higher than that of an organic solvent to be used for dissolving a polymer (but below the boiling point of water) (JP-2000-239152)
(II) a method of coating collected microspheres with an additive having a high melting point, followed by drying them under warming (JP-A-9-221417)

Microspheres thus obtained are used in the form of fine granules, suspensions, embedded type preparations, injections, adhesive preparations, etc. and can be administered orally or parenterally [intramuscular injection, subcutaneous injection, administration into blood vessel, percutaneous administration, administration via mucous membrane (buccally, vaginally, rectally, etc.)].

When the microspheres are used as an injection preparation or a suspension preparation (e.g., dry syrup for oral administration), they may preferably be prepared in the form of a liquid preparation by incorporating a dispersing agent (e.g., nonionic surfactants, polyoxyethylene castor oil derivatives, cellulose thickeners), or alternatively, the microsphere may be dispersed in an aqueous solution of a dispersing agent as mentioned above and an excipient such as an anti-moisture absorbent, an aggregation inhibitor (e.g., mannitol, sorbitol, lactose, glucose, xylitol, maltose, galactose, sucrose, dextran), and solidified by lyophilization, dried under reduced pressure, spray drying, etc., and the solidified preparation is dissolved in distilled water for injection when used.

The above injection preparation (including solidified ones) may further optionally be incorporated by isotonic agents (e.g., sodium chloride, glycerin, sorbitol, glucose, etc.), pH adjustors (e.g., carbonic acid, acetic acid, oxalic acid, citric acid, phosphoric acid, hydrochloric acid, sodium hydroxide or a salt of these acids, for example, sodium carbonate, sodium hydrogen carbonate, etc.), preservatives [e.g., p-hydroxybenzoic acid esters (methyl ester, ethyl ester, propyl ester, butyl ester), benzyl alcohol, chlorobutanol, sorbic acid, boric acid, etc.].

The apparatus for preparation of microspheres of the present invention, which is used for preparation of microspheres in a closed system, is intended for efficiently carrying out the method for preparation of microsphere of the present invention, and as an apparatus for carrying out Step (d-1) in the method for preparation of microsphere of the present invention, an apparatus comprising an emulsifying device, a microsphere storage tank and a cross flow filter as set up in the following manner is exemplified.
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank having a function of evaporation of organic solvent;
(iii) the microsphere storage tank, the cross flow filter and the emulsifying device are connected in such a manner that a part of the emulsion contained in the microsphere storage tank is introduced into the cross flow filter, and a liquid passing over the cross flow filter is returned to the microsphere storage tank while a filtrate filtered through the cross flow filter is introduced into the emulsifying device as an aqueous solution.

When a water-miscible solvent is used as an organic solvent having a boiling point lower than that of water, the microsphere storage tank does not necessarily have a function of evaporation of organic solvent, and hence, an apparatus comprising an emulsifying device, a microsphere storage tank and a cross flow filter as set up in the following manner may be used as an apparatus for carrying out Step (d-1).
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank;
(iii) the microsphere storage tank, the cross flow filter and the emulsifying device are connected in such a manner that a part of the emulsion contained in the microsphere storage tank is introduced into the cross flow filter, and a liquid passing over the cross flow filter is returned to the microsphere storage tank while a filtrate filtered through the cross flow filter is introduced into the emulsifying device as an aqueous solution.

On the other hand, as an apparatus for carrying out the method for preparation of microsphere of the present invention wherein Step (d-2) is employed without recycling a filtrate filtered through the cross flow filter as an aqueous solution, an apparatus comprising an emulsifying device, a microsphere storage tank and a cross flow filter as set up in the following manner may be used.
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank having a function of evaporation of organic solvent;
(iii) the microsphere storage tank and the cross flow filter are connected in such a manner that the emulsion contained in the microsphere storage tank is introduced into the cross flow filter, and a liquid passing over the cross flow filter is returned to the microsphere storage tank while a filtrate filtered through the cross flow filter is discharged outside of the apparatus.

When a water-miscible solvent is used as an organic solvent having a boiling point lower than that of water, an apparatus comprising an emulsifying device, a microsphere storage tank and a cross flow filter as set up in the following manner may be used as an apparatus for carrying out Step (d-2).
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank;
(iii) the microsphere storage tank and the cross flow filter are connected in such a manner that the emulsion contained in the microsphere storage tank is introduced into the cross flow filter, and a liquid passing over the cross flow filter is returned to the microsphere storage tank while a filtrate filtered through the cross flow filter is discharged outside of the apparatus.

In these apparatuses for preparation of microspheres, the emulsifying device may be various emulsifying devices as described for the above-mentioned method for preparation of microsphere, and it may be selected according to the purpose thereof, and may be either emulsifying devices for continuous emulsification or emulsifying devices for emulsification by batch-treatment. Namely, the emulsification procedure is not carried out continuously but the transfer of a medicament-containing polymer solution and an aqueous solution and/or a filtrate from the cross flow filter into the emulsifying device may be carried out intermittently.

In the apparatuses for preparation of microsphere of the present invention, the emulsification procedure is carried out in a small scale repeatedly or continuously, and hence, the capacity of the emulsifying device can be downsized much more as compared with cases where the total volume of microsphere is prepared at one time, and the capacity of the emulsifying device is preferably in the range of 1/10 to 1/1000 of the capacity of the microsphere storage tank.

Besides, the emulsifying device has a structure to which a medicament-containing polymer solution and an aqueous solution are designed to be transferred, and a medicament-containing polymer solution and an aqueous solution are transferred into the emulsifying device from a storage tank containing them respectively. When carrying out Step (d-1), the circulation process is initiated without having a storage tank for aqueous solution by transferring an aqueous solution obtained as a filtrate into the emulsifying device via the cross flow filter from the microsphere storage tank, where an aqueous solution is contained beforehand.

The emulsifying device may have a function of controlling the introduction speed of a medicament-containing polymer and an aqueous solution (including cases of recycling a filtrate). For example, when a filtrate is recycled as an aqueous solution, the emulsifying device may have a function of controlling the amount of the medicament-containing polymer solution to be transferred according to the filtration speed of the filtrate from the cross flow filter (the introduction speed of the aqueous solution into the emulsifying device), a function of transferring a specific amount of the medicament-containing polymer solution at the instant when the volume of the filtrate (i.e., the aqueous solution in the emulsifying device) reaches a specific amount, a function of transferring a specific amount of the medicament-containing polymer solution at the instant when the concentration of organic solvent in the filtrate reaches a specific level or below while continuously introducing a filtrate (i.e., the aqueous solution in the emulsifying device) into the emulsifying device, or a function of regularly introducing the medicament-containing polymer solution while the filtration speed of the filtrate from the cross flow filter (introduction speed of aqueous solution into the emulsifying device) is kept constant, etc.

The emulsifying device is coupled to the microsphere storage tank in such a manner that the resulting emulsion may be transferred into the microsphere storage tank.

For the continuous emulsification, the apparatus may be designed, for example, so that the medicament-containing polymer solution and the aqueous solution may be transferred into the emulsifying device through the upper side or the lateral side thereof to be emulsified, and the resulting emulsion is transferred into the microsphere storage tank through the lower side of the emulsifying device. Further, the apparatus may be designed, for example, so that the medicament-containing polymer solution and the aqueous solution may be transferred into the emulsifying device through the lower side or the lateral side thereof for emulsification, and the resulting emulsion is overflowed from the top of the emulsifying device and automatically transferred into the microsphere storage tank.

When the emulsification is carried out by batch-treatment, i.e., intermittently, if the filtrate filtered through the cross flow filter is recycled as an aqueous solution, it may be possible to transfer the emulsion from the emulsifying device into the microsphere storage tank by utilizing said filtrate flow.

The microsphere storage tank may be any ones being made of various materials as described for the method for preparation of microsphere as mentioned above, and the microsphere storage tank has various functions of evaporating the organic solvent as described for the method for preparation of microsphere as mentioned above.

In the method for preparation of microsphere of the present invention, the volume of the aqueous solution, being made up of a majority of the volume of the emulsion, is not increased even though the microsphere production scale is increased, and hence, the microsphere storage tank for carrying out this method may be downsized, and the volume of the microsphere storage tank necessary for industrial production of 1 kg of microspheres can be kept down to 10 to 100 liters or like.

The cross flow filter may be any commercially available ones as exemplified for the method for preparation of microsphere as described above.

The microsphere storage tank and the cross flow filter are connected in such a manner that the emulsion contained in the microsphere storage tank is introduced into the cross flow filter, and only a liquid passing over the membrane filter is returned into the microsphere storage tank, while a filtrate filtered through the cross flow filter is introduced into the emulsifying device or discharged from the apparatus.

In the pathway for introducing a filtrate filtered through the cross flow filter into the emulsifying device, a function of solvent evaporation for evaporating the organic solvent from the filtrate may be added, and for said additional solvent evaporation, a suitable device for evaporation of solvent may be separately equipped, or the function of evaporation of organic solvent to be used in the microsphere storage tank may be used.

Moreover, each pathway may, if necessary, have a function of transfer promotion for the transfer of the emulsion, filtrate, etc., and any transferring means such as tube pump, magnet pump, gear pump, centrifugal pump, diaphragm pump, etc. may be used.

When an organic solvent such as methylene chloride, which is often used in the microsphere preparation, is industrially used, it is necessary to use a closed manufacturing apparatus for preventing the diffusion thereof into the outside in view of environmental problems ("A law in regard to monitor of the discharging amount etc. of specific chemical substances into environment and improvement of control thereof" published on July 13, 1999 as well as by an Order of the Government issued on March 29, 2000), and further, the microsphere production in a closed system is essential in view of asepticization and prevention of contamination of bacteria for microsphere to be used as a drug, but since the present apparatus for preparation of microsphere is downsized, the closed system is easily achieved and hence, the present apparatus is excellent as an apparatus for the industrial production of microsphere.

When the filtrate is discharged outside of the apparatus and the organic solvent is evaporated off by an equipment of evaporation of organic solvent, the organic solvent is preferably recovered and recycled if necessary so as to prevent the diffusion into the atmosphere of the organic solvent in the filtrate and the evaporated organic solvent.

The organic solvent is recovered by a method of liquefying by cooling, a method of introducing into cold water, or a method of adsorbing to porous particles, etc. The adsorbing method is done with an apparatus of adsorbing with fibrous active carbon, a general purpose apparatus of recovering chlorocarbon exhaust gas, a small type apparatus of recovering chlorocarbon exhaust gas, an apparatus of recovering a low concentration of chlorocarbon exhaust gas, an apparatus of adsorbing with granular active carbon, a fluidized bed apparatus of adsorbing with spherical active carbon, or an apparatus of compression and condensation by deeply cooling (cf., Handbook for use of chlorocarbon, pp. 85-93). More specifically, commercially available apparatuses such as an apparatus of recovering and deodorizing of solvent "Ameig" manufactured by Kurimoto Ltd. and an apparatus for adsorbing and condensing a gas of a solvent in low concentration "Haloneater" manufactured by Toyobo Co., Ltd. may be used without modification

The present invention is illustrated in more detail by Examples and Reference Examples, but the present invention should not be construed to be limited thereto.

### (Example of apparatus for preparation of microsphere)

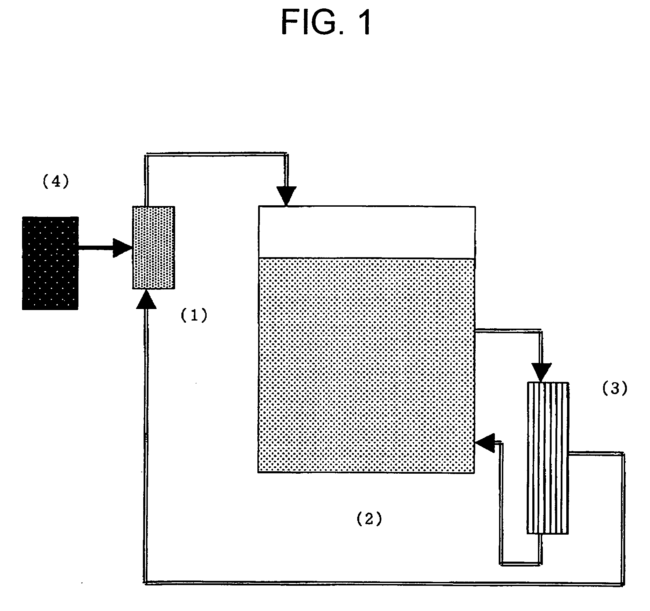

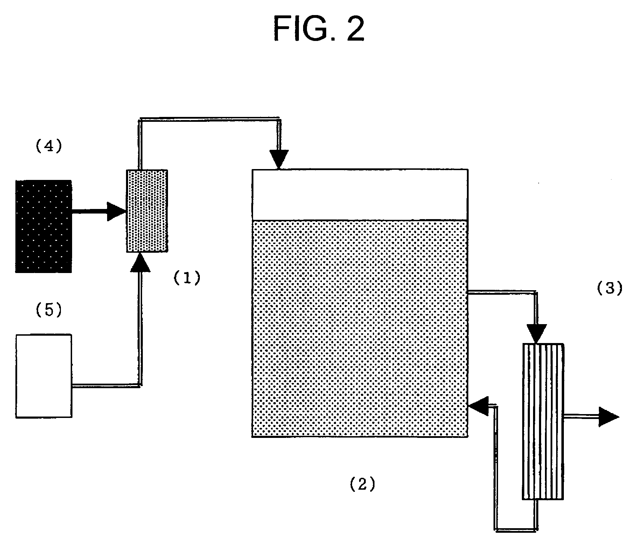

Examples of layouts of the apparatus, which may be possibly used for preparation of microsphere by the present method, are shown in Fig. 1 and Fig. 2.

In Fig. 1, a medicament-containing polymer solution is introduced from the storage tank for medicament-containing polymer solution (4) into the emulsifying device (1), while an aqueous solution is contained beforehand from the start in the microsphere storage tank (2), and introduced into the cross flow filter (3) therefrom. A liquid passing over the filter without being filtered is returned to the microsphere storage tank (2), and only a filtrate is introduced into the emulsifying device (1). After the emulsification, the resulting emulsion is transferred into the microsphere storage tank (2), and the emulsion, i.e., the content therein is introduced into the cross flow filter (3), and a liquid passing over the filter without being filtered is returned to the microsphere storage tank (2), and a filtrate is introduced into the emulsifying device (1), and said filtrate is emulsified together with the medicament-containing polymer solution introduced from the storage tank for medicament-containing polymer solution (4). The emulsion thus formed is transferred into the microsphere storage tank, and then the above procedures are repeated circularly.

In Fig. 2, a medicament-containing polymer solution and an aqueous solution are introduced into the emulsifying device (1) from the storage tank for medicament-containing polymer solution (4) and the storage tank for aqueous solution (5), respectively, and after the emulsification, the resulting emulsion is transferred into the microsphere storage tank (2), and the emulsion, i.e., the content therein is introduced into the cross flow filter (3), and a liquid passing over the filter without being filtered is returned to the microsphere storage tank (2), and a filtrate is discharged. Freshly, a medicament-containing polymer solution and an aqueous solution are introduced into the emulsifying device (1) from the storage tank for medicament-containing polymer solution (4) and the storage tank for aqueous solution (5), respectively, and emulsified. The emulsion thus obtained is transferred into the microsphere storage tank, and thereafter, the above procedures are repeated iteratively.

In the apparatuses of Fig. 1 and Fig. 2, the emulsifying device (1) may be either a device for continuous emulsification or a device for emulsification by non-continuous batch-treatment. Besides, the microsphere storage tank (2) may be one having a function of evaporation of organic solvent by gas blowing onto the liquid surface, or a function of evaporation of organic solvent by a hollow fiber membrane module, etc., but when the organic solvent having a low boiling point is miscible with water, then the microsphere storage tank (2) may be one having no such a function of evaporation of organic solvent.

### Example 1

(1) To leuprolide acetate (manufactured by BACHEM AG; drug content: 90.4 %) (1 g), and polylactic acid (average molecular weight: 17500; manufactured by Boehringer Ingelheim, RESOMER R202H) (9 g) are added methylene chloride (40 ml) and ethanol (10 ml), and the mixture is completely dissolved. This solution is evaporated to dryness by using a rotary evaporator heated at 60°C for 3 hours to remove the solvent, and the resultant is dried under reduced pressure overnight in a desiccator to give a solid solution. To this solid solution is added methylene chloride (20 g), and the mixture is made a completely clear solution.
(2) In the apparatus for preparation of microsphere as shown in Fig. 1 (employing emulsification by non-continuous batch-treatment, and a microsphere storage tank having a function of evaporation of organic solvent by a hollow fiber membrane module), the microspheres are prepared. That is, to the stainless-steel microsphere storage tank (closed tank; capacity: 20 liters; manufactured by M Technique, Inc.) having a stirrer (CLM-0.5SD) and a hollow fiber membrane module (NAGASEP flat type M60-600L-3600; effective area: 1.8 m²; manufactured by Nagayanagi Kogyo Kabushiki Kaisha) equipped therein is added previously a 0.1 % aqueous solution of polyvinyl alcohol (Gosenol EG-40; saponifying degree; 86.5-89.0 mole %; manufactured by The Nippon Synthetic Chemical Industry Co., Ltd.) (15 liters), and the mixture is stirred at 400 rpm. Further, a nitrogen gas is blown into the inside of the hollow fiber membrane module at a rate of 15 liters/minute. The aqueous polyvinyl alcohol solution in the tank is introduced via a tube pump (XX80EL000; manufactured by Millipore Corporation) to a cross flow filter (Prostak; membrane pore size: 0.65 µm, total membrane area: 0.332 m²; manufactured by Millipore Corporation) at a rate of 10 liters/minute, and the filtrate filtered through the cross flow membrane filter under a pressure of 0.03 - 0.05 MPa is flowed at a rate of 250 ml/minute into the emulsifying device (capacity: 350 ml; Clearmix CLM-1.5S; rotor: R4; screen: S 1.5-2.4; manufactured by M Technique, Inc.) via a tube pump (XX80EL000; manufactured by Millipore Corporation). On the other hand, the solution (22 ml) obtained in the above (1) is filled into a syringe, and injected into the emulsifying device in 2 ml portions over 2-3 seconds every 2 minutes. The emulsification is carried out at 16000 rpm, and the emulsion overflowed from the emulsifying device by the influx of the filtrate is introduced into the stainless-steel microsphere storage tank having a stirrer. The emulsification procedure is continued until one minute after the final injection of the solution obtained in the above (1), then 5 minutes after the final injection, the influx of the filtrate into the emulsifying device is stopped by stopping the tube pump at the filtrate side (while the circulation into the cross flow filter is continued). Then, a nitrogen gas is blown into the hollow fiber membrane module at room temperature for one hour at a rate of 15 liters/minute to remove the organic solvent from the emulsion.
(3) After the organic solvent is evaporated off, the tube pump at the filtrate side is restarted, and the obtained filtrate is discharged at a rate of 250 ml/minute. When the volume of the content within the microsphere storage tank becomes about 3 liters, purified water (12 liters) is added at a rate of 250 ml/minute from the upper part of the tank while the cross flow filtration is continued. Then, by continuing the filtration, the volume of the content within the microsphere storage tank is adjusted to about 1 liter. The content within the microsphere storage tank is transferred into a glass beaker, and purified water (1 liter) is added to the microsphere storage tank, and the inside of the cross flow filter is washed by circulation, and further the content within the microsphere storage tank is transferred into a beaker to collect the remaining microspheres. The recovery procedure of the remaining microspheres is repeated again, and the obtained microsphere suspension (about 3 liters) is centrifuged (2000 rpm, 10 minutes) to collect microspheres.
(4) The collected microspheres are transferred into a petri dish, and thereto is added a small amount of water, and the mixture is frozen at -40°C by a lyophilizer (RLE-100BS; manufactured by Kyowa Shinku Co.), and dried at 20°C under 0.1 Torr (13.3 Pa) for more than 15 hours to give lyophilized microsphere powders.

The average particle size of the lyophilized microsphere powder is measured by dispersing a suitable amount of the lyophilized microsphere powder in a diluted solution of polyoxyethylene sorbitan fatty acid ester (Tween 80; manufactured by Nikko Chemicals Co., Ltd.), and measured by a laser diffraction particle size analyzer (SALD-1100, manufactured by Shimadzu Corporation), and as a result, it was 4.9 µm.

The recovery rate, which is a percentage of the weight of the lyophilized microsphere powder to the total weight of the polylactic acid and leuprolide acetate to be used, was 79 %.

The lyophilized microsphere powder (5 mg) is dissolved in acetonitrile (1.5 mL). To the solution is added a 0.5 M aqueous sodium chloride solution (3.5 mL), and the mixture is subjected to centrifugation at 2,000 rpm for 10 minutes to separate the precipitates. To the resulting supernatant (200 µL) is added a mobile phase [26 % (v/v) acetonitrile/0.05 M potassium phosphate (pH 2.5), 800 µL], and the mixture is measured by HPLC apparatus [column packing: Nucleosil 100-5C18 (GL-science); column temperature: 40°C; flow rate: 1.0 ml/minute, wave length for detection: 280 nm], and based on a calibration curve previously prepared using a solution of leuprolide acetate in acetate buffer (pH 4.7), the content of leuprolide acetate in the microsphere particles is calculated. As a result, it was 9.13 %.

In 1,4-dioxane (for high performance liquid chromatography; manufactured by Katayama Chemical Inc.) (1 ml) containing bromoform (2.9 mg/ml; manufactured by Nacalai Tesque Inc.) is dissolved the microsphere powder (25 mg) to give a test sample solution. This test sample solution (2 µL) is measured with a gas chromatogram apparatus (the main body GC-14B, Integrator CR-7A, manufactured by Shimadzu Corporation) [column packing; Gaschropack 54 (manufactured by GL Science), column temperature: 160°C; the detector: FID; detection temperature: 170°C; injection temperature: 180°C; mobile gas: nitrogen gas; flow rate: 60 mL/minute; air: 40 kPa; H₂: 60 kPa], and based on a calibration curve previously prepared with a standard solution of methylene chloride in 1,4-dioxane containing bromoform (2.9 mg/ml), the concentration of the test sample liquid is estimated, and then in the light of the weight of microsphere particles used, the content of the methylene chloride in the microsphere particles is calculated. As a result, it was 1740 ppm.

### Example 2

The content in the microsphere storage tank is introduced into the cross flow filter at a rate of 6 liters/minute, and the filtrate is flowed into the emulsifying device at a rate of 120 ml/minute. The lyophilized microsphere powder is obtained in the same manner as in Example 1 except that the injection of the solution obtained in Example 1-(1) is carried out 2, 5, 8, 12, 16, 21, 26, 31, 37 and 43 minutes after the first injection thereof.

The average particle size as measured in the same manner as in Example 1 was 6.33 µm, and the recover rate was 78.8%. When calculating the content of leuprolide acetate contained in the microsphere particles in the same manner as in Example 1, it was 8.87 %. Then, when calculating the content of methylene chloride in the microsphere particles from the microsphere powder in the same manner as in Example 1, it was 702 ppm.

### Example 3

(1) To leuprolide acetate (manufactured by BACHEM AG; drug content: 90.4 %) (2.4 g) and polylactic acid (average molecular weight: 17500; manufactured by Boehringer Ingelheim, RESOMER R202H) (18.0 g) are added methylene chloride (80 ml) and ethanol (20 ml), and the mixture is completely dissolved. This solution is filtered through a filter having a membrane pore size of 0.22 µm (Durapore, GVWP), and evaporated to dryness by using a rotary evaporator heated at 60°C for 3 hours, and the resultant is dried under reduced pressure overnight in a desiccator to give a solid solution. To this solid solution is added methylene chloride (40 g), and the mixture is made a completely clear solution.
(2) In the apparatus for preparation of microsphere as shown in Fig. 1 (employing emulsification by non-continuous batch-treatment, and a microsphere storage tank having a function of evaporation of organic solvent by a hollow fiber membrane module), the microspheres are prepared. That is, to the stainless-steel microsphere storage tank (closed tank; capacity: 20 liters; manufactured by M Technique, Inc.) having a stirrer (CLM-0.5SD) and a hollow fiber membrane module (NAGASEP flat type M60-600L-3600; effective area: 1.8 m²; manufactured by Nagayanagi Kogyo Kabushiki Kaisha) equipped therein is added a 0.1 % aqueous solution of polyvinyl alcohol (Gosenol EG-40; saponifying degree; 86.5-89.0 mole %; manufactured by The Nippon Synthetic Chemical Industry Co., Ltd.) (15 liters), which is previously filtered through a filter having a membrane pore size of 0.22 µm (Durapore, GVWP), and the mixture is stirred at 400 rpm. Further, a nitrogen gas is blown into the inside of the hollow fiber membrane module at a rate of 25 liters/minute. The aqueous polyvinyl alcohol solution in the tank is introduced via a tube pump (XX80EL000; manufactured by Millipore Corporation) to a cross flow filter (Prostak; membrane pore size: 0.65 µm, total membrane area: 0.332 m²; manufactured by Millipore Corporation) at a rate of 10 liters/minute, and the filtrate filtered through the cross flow membrane filter under a pressure of 0.03 - 0.05 MPa is flowed at a rate of 250 ml/minute into the emulsifying device (capacity: 350 ml; Clearmix CLM-1.5S; rotor: R4; screen: S 1.5-2.4; manufactured by M Technique, Inc.) via a tube pump (XX8200115, manufactured by Millipore Corporation). On the other hand, the solution (22 ml) obtained in the above (1) is filled into a syringe, and injected into the emulsifying device in 2 ml portions over 2-3 seconds every 2 minutes. The emulsification is carried out at 16000 rpm, and the emulsion overflowed from the emulsifying device by the influx of the filtrate is introduced into the stainless-steel microsphere storage tank having a stirrer. The emulsification procedure is continued until one minute after the final injection of the solution obtained in the above (1), then 5 minutes after the final injection, the influx of the filtrate into the emulsifying device is stopped by stopping the tube pump at the filtrate side (while the circulation into the cross flow filter is continued). Then, a nitrogen gas is blown into the hollow fiber membrane module at room temperature for 2 hours at a rate of 25 liters/minute to remove the organic solvent from the emulsion.
(3) After the organic solvent is evaporated off, the tube pump at the filtrate side is restarted, and the obtained filtrate is discharged at a rate of 250 ml/minute. When the volume of the content within the microsphere storage tank becomes about 3 liters, purified water (12 liters) is added at a rate of 250 ml/minute from the upper part of the tank while the cross flow filtration is continued. Then, by continuing the filtration, the volume of the content within the microsphere storage tank is adjusted to about 1 liter. The content within the microsphere storage tank is transferred into a glass beaker, and purified water (1 liter) is further added to the microsphere storage tank, and the inside of the cross flow filter is washed by circulation, and further the content is transferred into a beaker to collect the remaining microspheres. The recovery procedure of the remaining microspheres is repeated again.
(4) The obtained microsphere suspension (about 3 liters) is transferred into a stainless-steel tray, and the mixture is frozen at -40°C by a lyophilizer (RLE-100BS; manufactured by Kyowa Shinku Co.), and dried at 20°C under 0.1 Torr (13.3 Pa) for about 40 hours to give lyophilized microsphere powder.

The average particle size as measured in the same manner as in Example 1 is 5.49 µm, and the recover rate was 74.7 %. When calculating the content of leuprolide acetate contained in the microsphere particles in the same manner as in Example 1, it was 10.05 %. Then, when calculating the content of methylene chloride in the microsphere particles from the microsphere powder in the same manner as in Example 1, it was 709 ppm.

### Example 4

The lyophilized microsphere powder (30.0 mg as leuprolide acetate, 298.5 mg as microsphere) is weighed and put into a glass vial (capacity: 5 ml, manufactured by West). To the vial is further added a 2 % aqueous solution of dextran 40 (manufactured by S & D Chemicals) (2.5 ml), which is previously filtered through a filter having a membrane pore size of 0.22 µm (Durapore, GVWP). After lightly stirring, the mixture is frozen with a lyophilizer (RL-100BS, manufactured by Kyowa Shinku Co.) at -40°C, and then dried at 20°C, 0.1 Torr (13.3 Pa) for about 18 hours to give lyophilized microsphere .

### Example 5

To the lyophilized microsphere obtained in Example 3 are added 0.1 % polyoxyethylene sorbitan fatty acid ester (Tween 80, manufactured by Nikko Chemicals Co., Ltd.), 0.5 % sodium carboxymethyl cellulose [Kicorate FTS-1, viscosity (neat 1 %): 30-50 mPa·s, manufactured by Nichirin Chemical Industries, Ltd.], 5 % aqueous D-mannitol solution (1.5 ml), and the microsphere is dispersed to give a dosage form.

### INDUSTRIAL APPLICABILITY

According to the present invention, a closed and downsized apparatus for preparation of microsphere can be made possible by using a cross flow filter during the production of microsphere by in-water drying method, so that the diffusion of an organic solvent into the atmosphere, which causes an environmental problem, can be prevented and microspheres having a high quality may be produced. Therefore; the present invention provides an extremely excellent method for industrial production of medicament-containing microsphere, and apparatuses to be used therefor.

## Claims

1. A method for preparation of microsphere, which comprises the following steps:
(a) emulsifying a medicament-containing polymer solution containing a medicament, a biocompatible and biodegradable hardly-water-soluble polymer and an organic solvent having a boiling point lower than that of water into an aqueous solution in an emulsifying device to form an emulsion wherein said medicament-containing polymer solution is dispersed in the aqueous solution;
(b) transferring the obtained emulsion into a microsphere storage tank;
(c) introducing a part of the emulsion from the microsphere storage tank into a cross flow filter;
(d-1)-i) returning a liquid passing over the filter to the microsphere storage tank;
(d-1)-ii) recycling a filtrate filtered from the above cross flow filter as an aqueous solution for Step (a), repeating Steps (a) to (d-1), and when the organic solvent having a boiling point lower than that of water is immiscible with water, then evaporating said organic solvent in the microsphere storage tank during this circulation process; or
(d-2)-i) returning a liquid passing over the cross flow filter to the microsphere storage tank;
(d-2)-ii) discharging a filtrate filtered from the above cross flow filter without recycling it as the aqueous solution for Step (a), repeating Steps
(a) to (d-2) with using a fresh aqueous solution, and when the organic solvent having a boiling point lower than that of water is immiscible with water, then evaporating said organic solvent in the microsphere storage tank during this circulation process;
(e) collecting microsphere in the microsphere storage tank after Step (d-1) or (d-2) is completed.

2. The method according to claim 1, wherein the medicament-containing polymer solution is one of the following ones:
(i) a solution in which a biocompatible and biodegradable hardly-water-soluble polymer and a medicament are dissolved in an organic solvent having a boiling point lower than that of water;
(ii) a suspension in which a biocompatible and biodegradable hardly-water-soluble polymer is dissolved in an organic solvent having a boiling point lower than that of water, and a medicament is suspended in the resulting polymer solution;
(iii) a dispersion in which a biocompatible and biodegradable hardly-water-soluble polymer is dissolved in an organic solvent having a boiling point lower than that of water, and an aqueous solution of medicament is dispersed in the resulting polymer solution;
(iv) a dispersion in which one of biocompatible and biodegradable hardly-water-soluble polymers is dissolved in an organic solvent having a boiling point lower than that of water, and a solution of the other biocompatible and biodegradable hardly-water-soluble polymer in the same organic solvent is dispersed in the resulting polymer solution, and further a medicament is dissolved or suspended in the dispersed polymer solution.

3. The method according to claim 1 or 2, wherein the emulsification of Step (a) is carried out continuously, and the resulting emulsion is transferred continuously into the microsphere storage tank.

4. The method according to claim 1 or 2, wherein the emulsification of Step (a) is carried out by batch-treatment, and the resulting emulsion in each batch is transferred individually into the microsphere storage tank.

5. The method according to any one of claims 1 - 4, wherein the organic solvent having a boiling point lower than that of water is immiscible with water, and the organic solvent having a boiling point lower than that of water is evaporated from an emulsion in the microsphere storage tank by warming, pressure reduction, blowing of a gas, evaporation with a hollow fiber membrane module, or a combination of these methods.

6. The method according to claim 5, wherein the organic solvent having a boiling point lower than that of water is evaporated from an emulsion in the microsphere storage tank by evaporation with a hollow fiber membrane module.

7. The method according to claim 6, wherein the organic solvent having a boiling point lower than that of water is a halogenated aliphatic hydrocarbon solvent.

8. The method according to any one of claims 1 to 4, wherein the organic solvent having a boiling point lower than that of water is miscible with water, and the evaporation of the organic solvent having a boiling point lower than that of water from an emulsion in a microsphere storage tank is not carried out.

9. The method according to claim 8, wherein the organic solvent having a boiling point lower than that of water is a water-miscible ketone solvent.

10. The method according to claim 8, wherein the medicament-containing polymer solution is one in which a medicament is suspended and a biocompatible and biodegradable hardly-water-soluble polymer is dissolved in a water-miscible organic solvent having a boiling point lower than that of water, and the aqueous solution is a uniform aqueous solution containing a solvent being immiscible with said water-miscible organic solvent but being miscible with water.

11. The method according to claim 1 or 2, wherein during Step (d-1) or Step (d-2), the filtration speed through the cross flow filter and the influx speed of the emulsion from the emulsifying device into the microsphere storage tank are controlled substantially the same so as to keep the volume of the emulsion in said tank substantially constant.

12. The method according to claim 4, the capacity of the microsphere storage tank is 10 to 1000 times of that of the emulsifying device for batch-treatment.

13. The method according to any one of claims 1 to 12, wherein the pore size of the membrane filter of the cross flow filter is in the range of 1/300 to 1/3 of the average particle size of the desired microsphere, and the filtration speed of the filtrate from the cross flow filter is adjusted to the range of 1/ 100 to 1/3 of the introduction speed of the emulsion into said cross flow filter.

14. The method according to claim 13, wherein the pore size of the membrane filter of the cross flow filter is within the range of 0.01 to 10 µm.

15. The method according to any one of claims 1 to 14, wherein the emulsification is carried out by a high-speed rotary homogenizer utilizing inner shear (liquid-liquid shear).

16. The method according to any one of claims 1 to 15, wherein the emulsification in Step (a) is carried out using the aqueous solution in a volume of 1 to 1000 times of that of the medicament-containing polymer solution.

17. The method according to claim 1, wherein Step (d-1) is employed.

18. The method according to claim 1, wherein Step (d-2) is employed.

19. The method according to any one of claims 1 to 18, wherein the biocompatible and biodegradable hardly-water-soluble polymer is a polyester of hydroxyfatty acid.

20. The method according to any one of claims 1 to 19, wherein the microspheres are collected by dead-end filtration, cross flow filtration or centrifugation, or a combination of these methods.

21. The method according to claim 17, wherein the medicament is recovered from the aqueous solution after the collection of the microspheres.

22. A method for preparation of lyophilized microsphere, which comprises preparing microspheres by the method as set forth in any one of claims 1 to 21, dispersing the microspheres thus obtained in an aqueous solution of an excipient if necessary, and then subjecting the resultant to lyophilization.

23. A lyophilized microsphere as prepared by the method as set forth in claim 22.

24. An apparatus for preparation of microsphere from a medicament-containing polymer solution containing a medicament, a biocompatible and biodegradable hardly-water-soluble polymer and an organic solvent having a boiling point lower than that of water, and an aqueous solution in a closed system, wherein an emulsifying device, a microsphere storage tank and a cross flow filter are set up in the following manner:
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank having a function of evaporation of organic solvent;
(iii) the microsphere storage tank, the cross flow filter and the emulsifying device are connected in such a manner that a part of the emulsion contained in the microsphere storage tank is introduced into the cross flow filter, and a liquid passing over the cross flow filter is returned to the microsphere storage tank while a filtrate filtered through the cross flow filter is introduced into the emulsifying device as an aqueous solution.

25. The apparatus according to claim 24, wherein the function for evaporation of organic solvent of the microsphere storage tank is a function of evaporation with a hollow fiber membrane module.

26. An apparatus for preparation of microsphere from a medicament-containing polymer solution containing a medicament, a biocompatible and biodegradable hardly-water-soluble polymer and an organic solvent having a boiling point lower than that of water, and an aqueous solution in a closed system, wherein an emulsifying device, a microsphere storage tank and a cross flow filter are set up in the following manner:
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank;
(iii) the microsphere storage tank, the cross flow filter and the emulsifying device are connected in such a manner that a part of the emulsion contained in the microsphere storage tank is introduced into the cross flow filter, and a liquid passing over the cross flow filter is returned to the microsphere storage tank while a filtrate filtered through the cross flow filter is introduced into the emulsifying device as an aqueous solution.

27. An apparatus for preparation of microspheres from a medicament-containing polymer solution containing a medicament, a biocompatible and biodegradable hardly-water-soluble polymer and an organic solvent having a boiling point lower than that of water, and an aqueous solution in a closed system, wherein an emulsifying device, a microsphere storage tank and a cross flow filter are set up in the following manner:
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank having a function of evaporation of organic solvent;
(iii) the microsphere storage tank and the cross flow filter are connected in such a manner that the emulsion contained in the microsphere storage tank is introduced into the cross flow filter, and a liquid passing over the cross flow filter is returned to the microsphere storage tank while a filtrate filtered through the cross flow filter is discharged outside of the apparatus.

28. The apparatus according to claim 27, wherein the function for evaporation of organic solvent of the microsphere storage tank is a function of evaporation with a hollow fiber membrane module.

29. An apparatus for preparation of microspheres from a medicament-containing polymer solution containing a medicament, a biocompatible and biodegradable hardly-water-soluble polymer and an organic solvent having a boiling point lower than that of water, and an aqueous solution in a closed system, wherein an emulsifying device, a microsphere storage tank and a cross flow filter are set up in the following manner:
(i) it has a structure by which a medicament-containing polymer solution and an aqueous solution can be introduced into the emulsifying device;
(ii) the emulsifying device and the microsphere storage tank are connected in such a manner that an emulsion obtained in the emulsifying device can be transferred into the microsphere storage tank;
(iii) the microsphere storage tank and the cross flow filter are connected in such a manner that the emulsion contained in the microsphere storage tank is introduced into the cross flow filter, and a liquid passing over the cross flow filter is returned to the microsphere storage tank while a filtrate filtered through the cross flow filter is discharged outside of the apparatus.

30. The apparatus according to any one of claims 24 to 29, wherein the capacity of the microsphere storage tank is in the range of 10 to 100 liters per 1 kg of the microspheres to be prepared in one production, as well as 10 to 1000 times of the capacity of the emulsifying device.

31. The apparatus according to any one of claims 24 to 30, which has a function of adjusting the filtration speed of the filtrate from the cross flow filter and the influx speed of the emulsion into the microsphere storage tank, and wherein the pore size of the membrane filter of the cross flow filter is in the range of 0.01 to 10 µm.
